# EUROPEAN PATENT APPLICATION

(11) **EP 4 541 883 A1**
(43) Date of publication of application: **23.04.2025**
(21) Application number: 23827120.9
(22) Date of filing: 15.06.2023
(51) Int. Cl.: C12N 5/071, C12Q 1/02, C12M 1/34

(54) **METHOD FOR PRODUCING CELL SUPPORT COMPOSITE, METHOD FOR PRODUCING DRUG EVALUATION DEVICE, DRUG EVALUATION METHOD AND DRUG EVALUATION DEVICE**

(30) Priority: 20.06.2022 JP 2022098744
(71) Applicant: Nikkiso Co., Ltd., Tokyo 150-6022 (JP)
(72) Inventor: TAKAHASHI Etsushi, Kanazawa-shi Ishikawa 920-0177 (JP); KITAGAWA Fumihiko, Kanazawa-shi Ishikawa 920-0177 (JP); ISHIGURO Naoki, Kobe-shi, Hyogo 650-0047 (JP); MORINAGA Gaku, Kobe-shi, Hyogo 650-0047 (JP); SAITO Asami, Kobe-shi, Hyogo 650-0047 (JP); TAKATANI Masahito, Kobe-shi, Hyogo 650-0047 (JP)
(74) Representative: Algemeen Octrooi- en Merkenbureau B.V.
(86) International application number: PCT/JP2023/022327
(87) International publication number: WO 2023/248934

(57) **Abstract**

A method for producing a cell support composite (10) includes: culturing renal cells (24) in an aggregated state (30); isolating the aggregate (30) into individual cultured cells (16); seeding the cultured cells (16) on a base (12) at a seeding density of 5×10⁴ cells/cm² or more and 18×10⁴ cells/cm² or less; and culturing the cultured cells (16) on the base (12) for a period of 12 hours or longer and 48 hours or shorter to form a monolayer film of the cultured cells (16).

## Description

### TECHNICAL FIELD

The present invention relates to a method for producing a cell support composite, a method for producing a drug evaluation apparatus, a drug evaluation method, and a drug evaluation apparatus.

### BACKGROUND ART

A drug administered to a living body is excreted from the blood through the kidney into urine after acting in vivo. This transportation of the drug from the blood into the urine is carried out by transporter proteins in renal cells. Hence, the renal cells are susceptible to nephrotoxicity of the drug. Particularly, renal proximal tubule epithelial cells (RPTECs) tend to be most affected by the drug in the kidney. Thus, it is important to use renal cells to evaluate the kinetics and nephrotoxicity of drugs in order to clarify the action of the drugs.

In developing an assay for evaluating the kinetics and nephrotoxicity of the drugs using renal cells, a large amount of the renal cells may be required. In this regard, Patent Literature 1, for example, discloses a culture technique of obtaining a sufficient number of renal proximal tubule epithelial cells by suppressing gene expression of the cell cycle control factors.

### RELATED-ART LITERATURE

### PATENT LITERATURE

Patent Literature 1: JP2011-50358

### SUMMARY OF INVENTION

### TECHNICAL PROBLEM

In conventional culture techniques, there has been a problem that the physiological functions of renal cells are deteriorated by culture. For this reason, it has been difficult to evaluate drugs with high accuracy in a drug evaluation assay having incorporated therein cells cultured by a conventional method.

The present invention addresses the issue described above, and a purpose thereof is to provide a technique for improving the accuracy of the drug evaluation assay using cells.

### SOLUTION TO PROBLEM

An embodiment of the present invention relates to a method for producing a cell support composite. This production method includes: culturing renal cells in an aggregated state; isolating the aggregate into individual cultured cells; seeding the cultured cells on a base at a seeding density of 5×10⁴ cells/cm² or more and 18×10⁴ cells/cm² or less; and culturing the cultured cells on the base for a period of 12 hours or longer and 48 hours or shorter to form a monolayer film of the cultured cells.

Another embodiment of the present invention relates to a method for producing a drug evaluation apparatus. This production method includes installing the cell support composite prepared by the method for producing a cell support composite of the aforementioned embodiment on a support portion, or installing a base on a support portion and seeding cultured cells on the base by the method for producing a cell support composite of the aforementioned embodiment, thereby preparing a cell support composite, and forming a first chamber and a second chamber that are aligned across the cell support composite, wherein at least one of the chambers contains a drug to be evaluated.

Another embodiment of the present invention relates to a method for producing a drug evaluation method. This method includes evaluating transportation of a drug by cultured cells included in the cell support composite using the drug evaluation apparatus obtained by the method for producing a drug evaluation apparatus of the aforementioned embodiment.

Another embodiment of the present invention relates to a drug evaluation apparatus. This apparatus includes a cell support composite produced by the method for producing a cell support composite of the aforementioned embodiment, a support portion that supports the cell support composite, and a first chamber and a second chamber that are aligned across the cell support composite, wherein at least one of the chambers contains a drug to be evaluated.

Optional combinations of the aforementioned constituting elements, and mutual substitution of constituting elements and implementations of the present invention between methods, apparatuses, systems, etc. may also be practiced as additional modes of the present invention.

### ADVANTAGEOUS EFFECTS OF INVENTION

According to the present invention, the accuracy of a drug evaluation assay using cells can be improved.

### BRIEF DESCRIPTION OF DRAWINGS

[Fig. 1] Fig. 1 is a drawing schematically illustrating a cell support composite according to an embodiment.
[Fig. 2] Fig. 2A to Fig. 2C are stepwise drawings of a method for producing the cell support composite and the method for producing a drug evaluation apparatus according to the embodiment.
[Fig. 3] Fig. 3A to Fig. 3C stepwise drawings of a method for producing the cell support composite and the method for producing a drug evaluation apparatus according to the embodiment.
[Fig. 4] Fig. 4A to Fig. 4E are drawings schematically illustrating an adoption example of the cell support composite.
[Fig. 5] Fig. 5 is a chart summarizing time-dependent changes in gene expression levels in cells that constitute an aggregate.
[Fig. 6] Fig. 6A to Fig. 6C are graphs illustrating time-dependent changes in gene expression levels in cells that constitute an aggregate.
[Fig. 7] Fig. 7A to Fig. 7C are graphs illustrating time-dependent changes in gene expression levels in cells that constitute an aggregate.
[Fig. 8] Fig. 8A to Fig. 8C are graphs illustrating time-dependent changes in gene expression levels in cells that constitute an aggregate.
[Fig. 9] Fig. 9 shows an optical microscope image of cells isolated from an aggregate on the day after re-seeding.
[Fig. 10] Fig. 10 is a graph illustrating TEER at each elapsed time since re-seeding.
[Fig. 11] Fig. 11A and Fig. 11B are graphs illustrating time-dependent changes in TEER at each seeding density.
[Fig. 12] Fig. 12 is a diagram showing changes in the expression level of the OAT1 gene over time.
[Fig. 13] Fig. 13A to Fig. 13F are diagrams showing changes in the expression level of OAT1 protein over time.
[Fig. 14] Fig. 14A and Fig. 14B are diagrams showing the expression levels of kidney-associated transporter proteins in cell membrane proteins, and Fig. 14C is a diagram showing changes over time in the expression level of kidney-related transporter proteins in all proteins.
[Fig. 15] Fig. 15A is a graph illustrating results of membrane transportation of Adefovir, and Fig. 15B is a graph illustrating results of membrane transportation of Metformin.

### DESCRIPTION OF EMBODIMENTS

Hereinafter, the invention will be described based on preferred embodiments with reference to drawings. The embodiments do not limit the scope of the invention but exemplify the invention. Not all of the features and the combinations thereof described in the embodiments are necessarily essential to the invention. Identical or like constituting elements, members, processes shown in the drawings are represented by identical symbols, and a duplicate description will be omitted as appropriate. The scales and shapes of the parts shown in the figures are defined for convenience's sake to make the explanation easy and shall not be interpreted limitatively unless otherwise specified. Terms like "first", "second", etc. used in the specification and claims do not indicate an order or importance by any means and are used to distinguish a certain feature from the others. Those of the members that are not material to the description of the embodiments are omitted in the drawings.

The present inventors examined a drug evaluation assay using cells and came to understand as follows. That is, renal cells such as renal proximal tubule epithelial cells isolated from the kidney (primary cultured cells) are dedifferentiated by elimination of the in vivo environment or by a culture environment such as two-dimensional culture on a Petri dish, and the functions are gradually deteriorated or lost. For this reason, a simple culture of the renal cells only results in the proliferation of cells with insufficient physiological functions. There is a possibility that the cells with insufficient physiological functions may not exhibit normal transportation of medications or normal response to the toxicity of medications. Therefore, the use of renal cells for drug discovery research has been stalled.

The present applicant has found a technique for generating cultured cells with greatly improved renal physiological functions by culturing renal cells that have lost physiological functions in an aggregated state (International Application No. PCT/JP2018/011392). It is expected that the incorporation of the cells obtained with this technique into a drug evaluation assay will allow drug evaluation to be performed with high accuracy.

In drug evaluation assays, and especially drug kinetic evaluation of reabsorption and excretion of drugs in the kidney (transporter activity measurement), it is common to evaluate membrane transportation using a Transwell. If aggregates of cultured cells are used as they are, a uniform monolayer film of the cells cannot be formed on a cell culture insert of the Transwell (hereinafter referred to as the insert, as appropriate). Unless a monolayer film of cultured cells is obtained, it is difficult to accurately perform drug kinetic evaluation using the Transwell. Therefore, it is necessary to treat the aggregates with proteolytic enzymes to isolate them into individual cultured cells and then seed the isolated cultured cells on the insert.

However, when isolated cultured cells are subjected to two-dimensional culture on an insert, there is a risk that physiological functions enhanced by aggregation culture (three-dimensional culture) will deteriorate again. Besides, in a case where the cultured cells are seeded at a low density on the insert, it takes a long time until a monolayer film without gaps is formed. This makes physiological functions increasingly prone to deterioration. It is therefore conceivable to seed cultured cells without gaps on the insert in an aim to shorten the time from seeding of cultured cells to formation of a monolayer film. However, the present inventors have confirmed that even with such high-density seeding of cultured cells, a monolayer film with substantially no drug leakage, which is suitable for pharmacokinetic evaluation, cannot be obtained. For this reason, development of a drug evaluation assay using a monolayer film of cells having good physiological functions is desperately needed in drug discovery research.

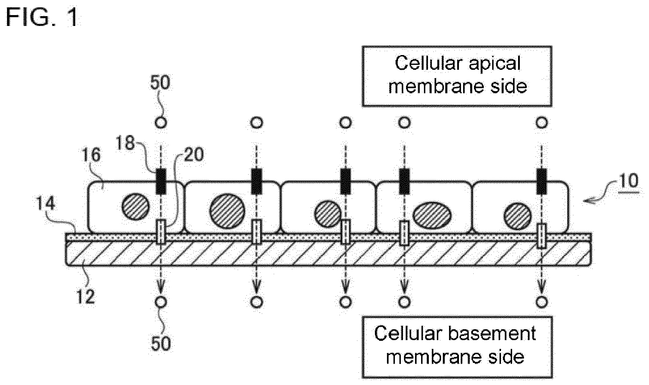

In this background, the present inventors have found a technique for forming a monolayer film of cultured cells on a base while suppressing deterioration of physiological functions enhanced by agglutination culture. The embodiment has been devised based on such contemplation. Fig. 1 is a drawing schematically illustrating a cell support composite 10 according to the embodiment. The cell support composite 10 includes a base 12 and a monolayer of cultured cells 16. The cell support composite 10 of the present embodiment also includes a coating agent layer 14.

### (Base)

The base 12 is formed from, for example, an artificial material. The base 12 has permeability to water and various ions. The base 12 also has permeability to sugars, low-molecular-weight proteins, etc. A drug 50 present on the cellular apical membrane side passes through the cell support composite 10 through a first transporter 18 on the cellular apical membrane side, a second transporter 20 on the cellular basement membrane side, which are provided in the cultured cells 16 and through the base 12 to reach the cellular basement membrane side. Transportation of the drug 50 by the cultured cells 16 includes not only transfer of the drug 50 present on the cellular apical membrane side to the cellular basement membrane side but also transfer of the drug 50 present on the cellular basement membrane side to the cellular apical membrane side.

The base 12 is provided, for example, with holes to make the base 12 permeable to various substances. The holes provided to the base 12 preferably have an average pore size of 5 µm or less. By adjusting the average pore size to 5 µm or less, the risk of the cultured cells 16 passing through the base 12 can be reduced. As such a base 12, a Transwell insert (Corning Inc.; average pore size 0.4 µm or 3.0 µm) can, for example, be used.

The base 12 having no permeability to various substances may also be used. In this case, the drug 50 present on the cellular apical membrane side is incorporated, through the first transporter 18 of the cultured cells 16, inside the cultured cells 16. Within a short period from the start of use, only a small amount of the drug 50 moves through the second transporter 20 of the cultured cells 16 towards the cellular basement membrane side. Thus, deformation does not occur in the layer of the cultured cells 16. When a long period elapses, the amount of transfer of the drug 50 through the second transporter 20 increases, but the drug 50 cannot pass through the base 12. The layer of the cultured cells 16 is thus lifted up to form a dome.

Materials that constitute the base 12 are not particularly limited, but examples thereof include polystyrene, polycarbonate (PC), polyester (PET), a polyester-based polymer alloy (PEPA), an ethylene-vinyl alcohol copolymer (EVOH), polyethylene, polysulfone (PSf), and polyethersulfone (PES).

### (Cultured Cell)

The cultured cells 16 adhere to the base 12. The cultured cells 16 of the present embodiment are fixed to the base 12 with a coating agent layer 14. In a case where the coating agent layer 14 is omitted, the cultured cells 16 adhere directly to the base 12. As described above, the cultured cells 16 have the first transporter 18 that are positioned on the cellular apical membrane side and the second transporter 20 that are positioned on the cellular basement membrane side.

The cultured cells 16 are prepared by culturing renal cells in a state of being non-adherent to a culture vessel. The cultured cells 16 need to maintain the physiological functions to be used for drug evaluation. On the other hand, when renal cells are cultured in an environment different from an in vivo environment, they are dedifferentiated, and the physiological functions deteriorate. In contrast, the deteriorated physiological functions of renal cells can be restored by culturing the renal cells in a state of being non-adherent to a culture vessel. Specifically, when renal cells are cultured in a state of being non-adherent to a culture vessel, the renal cells form aggregates during the culture period. For example, the aggregates are formed on the first day of culture (that is, within 24 hours). The physiological functions of renal cells can then be restored by culturing the renal cells in an aggregated state for at least a part of the period. A method for culturing cells will be explained in detail later. The aggregates also include organoids.

The renal cells on which the cultured cells 16 are based include renal cells derived from a tissue. They also include renal cells derived from stem cells such as iPS cells, ES cells, or Muse cells. In addition, the renal cells include, for example, at least one of epithelial cells from a proximal tubule system, a distal tubule system, and a collecting duct system. More specifically, examples of the renal cells include human renal proximal tubule epithelial cells, human distal tubule epithelial cells, and human collecting duct epithelial cells, all of which are collected and isolated from the kidney. Examples thereof also include renal proximal tubule epithelial cells, distal tubule epithelial cells, and collecting duct epithelial cells obtained by inducing differentiation from human iPS cells, human ES cells, or human Muse cells. The renal cells are preferably renal proximal tubule epithelial cells. The renal cells also include immortalized cells and established cell lines (such as HK-2 cells) of the above-described renal cells, transformed cells obtained by introducing a gene into renal cells to express a protein such as a specific transporter, and renal progenitor cells. In addition, cells derived from other animal species (such as MDCK cells, LLC-PK1 cells, and JTC-12 cells) can also be used as the renal cells in place of renal cells derived from humans.

The cultured cells 16 form a confluent monolayer on a base 12 without substantial multilayering. The "monolayer" is preferably a layer in which multilayering of the cultured cells 16 does not occur at all. However, the "monolayer" can also include a structure that is partially multilayered to the extent that a decrease in efficiency of the substance transfer due to multilayering does not cause a problem (substantial monolayer). In addition, "confluent" denotes a state in which the area of a region occupied by the cultured cells 16 in an observed image preferably accounts for 100% of the total area of the observed image. For example, the term "confluent" means a state in which the proportion of the area occupied by the cells in the entire culture surface of the base 12 is 100%, that is, the cells have proliferated fully over the culture surface without any gaps. However, "confluent" can also include a state in which some gaps exist to the extent that the occurrence of concentration-dependent substance transfer in the gaps between the neighboring cells does not cause a problem (substantial confluency). Whether it is a monolayer or not and confluent or not can be easily determined by those skilled in the art.

### (Coating Agent Layer)

The coating agent layer 14 is formed from a coating agent. The coating agent layer 14 covers at least a part of the base 12. The coating agent layer 14 is adhered to the surface of the base 12 and thus fixed to the base 12. The coating agent contains one or more adhesive molecules selected from the group consisting of laminin molecules, fragments of laminin molecules, a basement membrane matrix mixture, fragments of a basement membrane matrix mixture, collagen molecules, and fragments of collagen molecules. The laminin molecules, basement membrane matrix mixture, collagen molecules and fragments of these may each be used singly, or two or more kinds thereof may be used in a mixed manner. The coating agent layer 14 can contain these adhesive molecules to make it easier to form a monolayer film of the cultured cell 16. The coating agent may also contain other adhesion proteins such as gelatin, fibronectin, and fragments of these, in addition to or in place of the adhesion molecules described above.

### (Laminin Molecule)

A laminin molecule has a hetero-trimer structure having one each of an α-chain, a β-chain, and a γ-chain. At present, five kinds of α-chins, three kinds of β-chains, and three kinds of γ-chains have been identified. Laminin molecules are known to form at least 12 kinds of isoforms through combinations of these. For example, the laminin molecule is selected from one or more of laminin 111, laminin 211, laminin 221, laminin 311, laminin 332, laminin 421, laminin 511, and laminin 521.

Laminin molecules also encompass modified bodies of laminins (modified laminins) obtained by adding predetermined modifying groups into one or more sites of the above-described isoforms. The modified bodies also encompass gene recombinants, that is, proteins produced by introducing mutation into proteins obtained from recombinant genes, partial proteins of gene recombinants, and proteins having peptides derived from gene recombinants. The modifying group of the modified laminin is, for example, a growth factor binding molecule or a cell-adhesive molecule.

The concentration of laminin molecules in the coating agent and the amount of adhesion of laminin molecules to the base 12 are adjusted as appropriate so that the cell support composite 10 can maintain its performance during the period of practical use. The amount of adhesion of laminin molecules can be controlled by adjusting the concentration of laminin molecules in the coating agent. The amount of adhesion of laminin molecules to the base 12 can be measured using a method known to those skilled in the art.

An example of a fragment of laminin molecule is a modified body of an E8 region (denoted as laminin ***-E8) containing a cell adhesive site (integrin binding site) of domain I in a laminin molecule (full-length laminin). Examples of such a modified body include laminin 111-E8, laminin 211-E8, laminin 421-E8, laminin 511-E8 and laminin 521-E8. Not only a modified body of the E8 region but also a laminin peptide having cell adhesion activity, or a product obtained by peptide synthesis of only cell-active sites can be used as the fragment of the laminin molecule. Examples of such a laminin peptide include a YIGSR-containing peptide, a PDSGR-containing peptide, an RYVVLPR-containing peptide, an RGD-containing peptide, a KAFDITYVRLKF-containing peptide, an IKVAV-containing peptide, and an LRE-containing peptide. The size of the fragment of the laminin molecule is not particularly limited.

The laminin molecule and the fragment thereof may each be used in the form of a mixture of a plurality of isoforms. The laminin molecule and the fragment thereof may be used in a mixed manner. Alternatively, a base 12 may be coated with a plurality of coating agents that contain different kinds of the laminin molecules and/or fragments thereof, and a plurality of coating agent layers 14 that contain different kinds of laminin may be layered.

### (Basement Membrane Matrix Mixture)

The basement membrane matrix mixture is a mixture of extracellular matrix proteins extracted from mouse sarcoma. The basement membrane matrix mixture contains laminin, collagen IV, and entactin as main constituent components. An example of the basement membrane matrix mixture is Matrigel (R) (Corning Inc.). Matrigel refers to a soluble basement membrane matrix extracted from Engelbreth-Holm-Swarm (EHS) mouse sarcoma, which is rich in extracellular matrix proteins. Matrigel also includes, in addition to conventional Matrigel containing a growth factor, Matrigel with a reduced growth factor compared to the conventional Matrigel (Growth Factor Reduced Matrigel Matrix).

The concentration of the basement membrane matrix mixture in the coating agent and the amount of adhesion of the basement membrane matrix mixture to the base 12 are adjusted as appropriate so that the cell support composite 10 can maintain its performance during the period of practical use. The amount of adhesion of the basement membrane matrix mixture can be controlled by adjusting the concentration of the basement membrane matrix mixture in the coating agent. The amount of adhesion of the basement membrane matrix mixture to the base 12 can be measured using a method known to those skilled in the art.

A fragment of the basement membrane matrix mixture is a mixture of at least one of a fragment of laminin, a fragment of collagen IV, or a fragment of entactin. Also, the basement membrane matrix mixture and fragments thereof may each be used in a mixture of a plurality of kinds. Besides, the basement membrane matrix mixture and fragments thereof may be used in a mixed manner. Alternatively, a base 12 may coated with a plurality of coating agents that contain different kinds of basement membrane matrix mixtures and/or fragments thereof, and a plurality of coating agent layers 14 that contain different kinds of mixtures may be layered.

### (Collagen Molecule)

Examples of collagen molecules include collagen I and collagen IV. They are commercially available from, for example, Nitta Gelatin Inc. The concentration of collagen molecules in the coating agent and the amount of adhesion of collagen molecules to the base 12 are adjusted as appropriate so that the cell support composite 10 can maintain its performance during the period of practical use. The amount of adhesion of collagen molecules can be controlled by adjusting the concentration of collagen molecules in the coating agent. The amount of adhesion of collagen molecules to the base 12 can be measured using a method known to those skilled in the art.

The collagen molecule and fragments thereof may each be used in a mixture of a plurality of kinds. The collagen molecule and fragments thereof may also be used in a mixed manner. Alternatively, a base 12 may be coated with a plurality of coating agents that contain different kinds of collagen molecules and/or fragments thereof, and a plurality of coating agent layers 14 that contain different kinds of collagen may be layered.

### (Method for Producing Cell Support Composite and Method for Producing Drug Evaluation Apparatus)

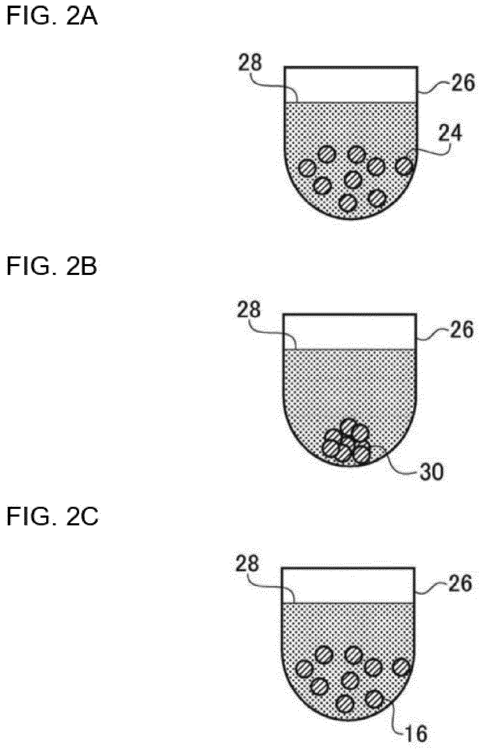

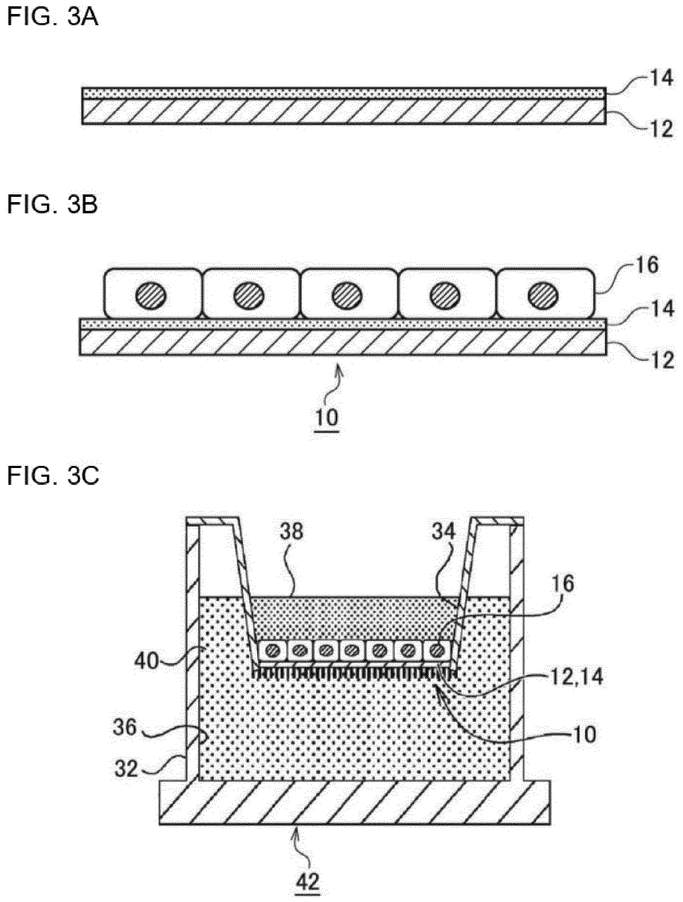

Fig. 2A to Fig. 2C and Fig. 3A to Fig. 3C are stepwise drawings of a method for producing the cell support composite 10 and the method for producing a drug evaluation apparatus 42 (drug evaluation module) according to the embodiment. Fig. 3A and Fig. 3B illustrate only a portion of the base 12.

The method for producing the cell support composite 10 according to the present embodiment includes: culturing renal cells 24 in the aggregate 30, isolating the aggregate 30 into individual cultured cells 16, seeding the cultured cells 16 on the base 12 at a predetermined seeding density, and culturing the cultured cells 16 on the base 12 for a predetermined period, thereby forming a monolayer film of the cultured cells 16. The method for producing the drug evaluation apparatus 42 according to the present embodiment includes: by installing the cell support composite 10 prepared by the method for producing the cell support composite 10 according to the present embodiment on the support portion 32, or by installing the base 12 on the support portion 32, and seeding the cultured cells 16 on the base 12 by the method for producing the cell support composite 10 according to the present embodiment to prepare the cell support composite 10, thereby forming the first chamber 34 and the second chamber 36 that are aligned across the cell support composite 10, wherein at least one of the chambers contains the drug 50 to be evaluated.

As illustrated in Fig. 2A as an example, renal cells 24 are seeded into a culture vessel 26. A culture solution 28 is added to the culture vessel 26. The culture vessel 26 is not particularly limited as long as it enables the renal cells 24 to be cultured in suspension. An example of the culture vessel 26 includes a U-bottom or V-bottom 96-well plate, a U-bottom 384-well plate, a microwell plate having a larger number of wells, a spinner flask, a Petri dish, a hollow fiber, a culture bottle, a culture bag, a micro flow channel, and a culture tank.

The culture vessel 26 may be a high-density spheroid preparation plate. This makes it possible to prepare the aggregates 30 in large quantities. Examples of the high-density spheroid preparation plate include an ELPLASIA (R) plate (Corning Inc.) and EZSPHERE (R) plate (AGC TECHNO GLASS Co., Ltd.). For example, the ELPLASIA plate is available in types such as a 6-well plate, a 24-well plate, a 96-well plate, and a 384-well plate. The bottom area of the wells varies according to the number of wells. The number of the aggregates 30 prepared varies according to the size of this bottom area. The aggregates prepared on the high-density spheroid preparation plate may be subsequently subjected to shaking suspension culture. In an example of shaking suspension culture, aggregates are contained in a dish or plate that has been subjected to a treatment that makes the dish or plate non-adherent to cells, and this dish, etc. is placed on a shaker to culture the aggregates. A reciprocating shaker or a turning shaker can be used as a shaker.

The culture solution 28 is not particularly limited as long as it enables the renal cells 24 to be cultured and can be selected from known culture media as appropriate, according to the kind of cells to be cultured, etc. In the case of culturing proximal tubule cells, for example, REGM (Lonza Group AG), EpiCM (ScienCell Research Laboratories, Inc.), and KeratinocyteSFM (Life Technologies Corp.) can be used. A commercially available tubule cell culture medium, epithelial cell culture medium, or vascular endothelial cell culture medium can also be used.

The renal cells 24 are then cultured. The renal cells 24 are cultured in a state of being non-adherent to the surface of the culture vessel 26. Therefore, as illustrated in Fig. 2B, aggregates 30 of the renal cells 24 are formed during the culture period. The cultured cells 16 that exhibit better physiological functions can be obtained by culturing the renal cells 24 in the aggregate 30. The size of the aggregates 30 is, for example, about 100 µm or more and about 350 µm or less. The size of the aggregate 30 is defined as the maximum width of the aggregate 30. That is, the size of the aggregate 30 is the length of the longest straight line among all straight lines that connect two points on the outer periphery of the aggregate 30.

The size of the aggregate 30 can be controlled by adjusting the number of cells to be seeded into the culture vessel 26. For example, in a case where a well plate is used as the culture vessel 26, the number of renal cells 24 seeded per well is preferably 500 or more and 5,000 or less. When cells are cultured in a well plate, one aggregate 30 is formed in one well. Therefore, when the number of renal cells 24 seeded per well is 500 or more and 5,000 or less, the number of renal cells that constitute the aggregates 30 is 500 or more and 5,000 or less. The size of the aggregate 30 is 100 µm or more and 350 µm or less when the number of constituent cells is 500 or more and 5,000 or less. By adjusting the number of constituent cells of the aggregates 30 to 500 or more or the size to 100 µm or more, it becomes easier to avoid suctioning of the aggregates 30 along with the old culture solution at the time of medium exchange or the like. Furthermore, by adjusting the number of constituent cells to 5,000 or less, or the size to 350 µm or less, the cultured cells 16 having better physiological functions can be obtained.

In a case where it is desired to form a larger number of aggregates 30, it is preferable to culture cells using a vessel such as a Petri dish or a spinner flask. In this case, the size of the aggregates 30, i.e., the number of renal cells 24 that constitute one aggregate 30, can be controlled by adjusting the cell density in the vessel. For example, in a case where the aggregates 30 having a size of about 100 µm or more and about 350 µm or less or containing 500 or more and 5,000 or less constituting cells are formed on a 60-mm Petri dish (Sumitomo Bakelite Co., Ltd.), the cell density in the Petri dish is adjusted to be 1,500 cells/cm² or more and 15,000 cells/cm² or less. Then, the renal cells 24 are subjected to stationary culture, shaking culture, or stirred culture, and, thereby, a plurality of aggregates 30 having the desired size or the desired number of constituent cells can be formed all at one time.

Collagen I may be added to the culture solution 28. Collagen I has a function of causing renal cells 24 to adhere to one another. Therefore, culturing the renal cells 24 in a culture solution 28 containing collagen I makes it easier to form the aggregates 30. Collagen I added is preferably full-length collagen I but may also be the α1 chain or α2 chain that constitute collagen I, or a collagen peptide obtained by fragmenting each chain. The animal species from which collagen I is obtained is not particularly limited, and not only collagen I derived from humans but also collagen I derived from other animals can be used.

It is preferable that the culture vessel 26 has been subjected to a treatment that makes the vessel non-adherent to cells or is formed from a material non-adherent to cells. As a result, aggregates 30 can be formed more easily. Examples of the treatment for non-adherence to cells include a hydrogel coating treatment for non-adherence to cells, an MPC (2-methacryloyloxyethyl phosphorylcholine) coating treatment, a PROTEOSAVE (R) SS coating treatment, and a mirror surface polishing treatment on the vessel surface. Examples of materials non-adherent to cells include glass. Examples of materials non-adherent to cells also include polymer materials such as low-density polyethylene, a medium-density polyethylene, polyvinyl chloride, a polyethylene-vinyl acetate copolymer, a poly(ethylene-ethyl acrylate) copolymer, a poly(ethylene-methacrylate) copolymer, a poly(ethylene-vinyl acetate) copolymer, and a mixture of two or more kinds of these polymers.

The culture period for the renal cells 24 is preferably 5 days or longer. Further, the culture period is preferably 90 days or shorter (that is, 2,160 hours or shorter) and, more preferably, 14 days or shorter (that is, 336 hours or shorter). By adjusting the culture period to 5 days or longer, cultured cells 16 in a state in which physiological functions are expressed at a higher level can be obtained easily. In addition, by adjusting the culture period to 90 days or shorter, cultured cells 16 in a state in which physiological functions are expressed at a higher level can be obtained easily. Besides, by adjusting the culture period to 14 days or shorter, the cultured cells can be easily isolated from the aggregates. The culture conditions are, for example, 37°C and 5% CO₂. It is preferable that during the culture period, the culture solution 28 is exchanged regularly. For example, the culture solution 28 is exchanged every two days.

Subsequently, as illustrated in Fig. 2C, the aggregates 30 are isolated into individual cultured cells 16. Isolation of the cultured cells 16 from the aggregates 30 can be carried out by enzymatically treating the aggregates 30 using Accumax, trypsin/EDTA, Accutase, collagenase, EDTA, TrypLE Select, or the like. In one example of the enzyme dispersion treatment, incubation at 37°C for 10 minutes or longer and pipetting with a micropipette are applied. As a result, a cell suspension in which single cultured cells 16 are dispersed can be obtained. The concentration of the enzyme used in the treatment can be set as appropriate according to the kind of cultured cells 16. However, in a case where the enzyme concentration is high, isolation of the cultured cells 16 is made easy, while there is a high risk that the cell surface proteins of the cultured cells 16 may be damaged. Accordingly, it is desirable that the enzyme concentration is as low as possible.

Furthermore, as illustrated in Fig. 3A, at least a part of the base 12 is coated with a coating agent. As a result, a coating agent layer 14 is formed on the surface of the base 12. Preparation and isolation of the cultured cells 16 and coating with the coating agent can be carried out independently of each other. That is, whichever of the two steps may be carried out first, or the two steps may be carried out in parallel.

As illustrated in Fig. 3B, the cultured cells 16 are seeded on the base 12. For example, the base 12 can be coated with the cell suspension obtained by the enzymatic dispersion treatment, thereby seeding the cultured cells 16 on the base 12. Then, the cultured cells 16 are cultured on the base 12, thereby forming a monolayer film of the cultured cells 16.

The cultured cells 16 are seeded on the base 12 at a seeding density of 5×10⁴ cells/cm² or more and 18×10⁴ cells/cm² or less. For example, in the case of using a cell culture insert for a 24-well plate with a seeding area of 0.336 cm² as the base 12, the cultured cells 16 are seeded on the base 12 at a seeding density of 2×10⁴ cells/well or more and 6×10⁴ cells/well or less. A cell culture insert for a 12-well plate with a seeding area of 1.131 cm² can also be used as the base 12.

In addition, the cultured cells 16 are cultured on the base 12 for a period of 12 hours or longer and 48 hours or shorter. The cultured cells 16 are cultured using, for example, REGM (Lonza, Inc.) as a culture solution under conditions of 37°C and 5% CO₂. The cultured cells 16 proliferate up to a confluent state on the base 12, and then the confluent state is maintained. Through the above steps, the cell support composite 10 can be obtained.

The seeding density of 5×10⁴ cells/cm² or more and 18×10⁴ cells/cm² or less and the culture period of 12 hours or longer and 48 hours or shorter can provide a monolayer film with substantially no drug leakage comprised of the cultured cells 16 that retain good physiological functions. More preferably, the cultured cells 16 are seeded on the base 12 at a seeding density of 5×10⁴ cells/cm² or more and 12×10⁴ cells/cm² or less. In the case of a cell culture insert with a seeding area of 0.336 cm², the cultured cells 16 are seeded on the base 12 at a seeding density of 2×10⁴ cells/well or more and 4x10⁴ cells/well or less. As a result, it is easier to extend the period during which the monolayer film of the cultured cells 16 is maintained properly, i.e., the period during which the cell support composite 10 can be used for drug evaluation.

Subsequently, as illustrated in Fig. 3C, the cell support composite 10 is installed on the support portion 32. The drug evaluation apparatus 42 of the present embodiment adopts the mode of Transwell. Therefore, the base 12 is a cell culture insert, and the support portion 32 is a well of a well plate. The insert as the base 12 is contained in the well as the support portion 32. As a result, the space in the well is divided into two by the cell support composite 10 to thereby form a first chamber 34 and a second chamber 36 that are aligned across the cell support composite 10.

The first chamber 34 is disposed above the cell support composite 10, i.e., on the monolayer film side of the cells, and the second chamber 36 is disposed below the cell support composite 10, i.e., on the base 12 side. Through the above steps, the drug evaluation apparatus 42 including the cell support composite 10, the support portion 32, the first chamber 34, and the second chamber 36 is obtained. In the explanation described above, the cell support composite 10 is prepared by seeding the cultured cells 16 on the base 12 before the base 12 is installed on the support portion 32, but the cell support composite 10 may alternatively be prepared by seeding the cultured cells 16 on the base 12 after the base 12 has been installed on the support portion 32.

At least one of the first chamber 34 or the second chamber 36 contains the drug 50 to be evaluated. As an example, a liquid obtained by adding the drug 50 to an equilibrium salt solution, such as HBSS buffer, is contained in at least one of the chambers. Fig. 3C illustrates the drug evaluation apparatus 42 in which a first liquid 38 is contained in the first chamber 34 while a second liquid 40 is contained in the second chamber 36. The drug 50 is added at least one of the first liquid 38 or the second liquid 40. The drug 50 can be in contact with the cultured cells 16 in each of a state in which it is contained in the first chamber 34 and a state in which it is contained the second chamber 36.

### (Drug Evaluation Method)

The drug evaluation method according to the present embodiment includes evaluating the transportation of the drug 50 by the cultured cells 16 included in the cell support composite 10 using the drug evaluation apparatus 42, obtained by the production method described above. This indicates that the drug evaluation apparatus 42 can evaluate intake and excretion of the drug 50 with the first transporter 18 and the second transporter 20. The evaluation also allows for prediction of an impact of the drug 50 on the kidneys.

For example, the ability of the first transporter 18 to intake the drug 50 can be evaluated by adding the drug 50 to the first liquid 38 that is positioned on the cellular apical membrane side of the cultured cells 16 and then measuring the amount of the drug 50 in the cultured cells 16 after a predetermined period. Further, the ability of the second transporter 20 to intake the drug 50 can be evaluated by adding the drug 50 to the second liquid 40 that is positioned on the cellular basement membrane side of the cultured cells 16 and then measuring the amount of the drug 50 in the cultured cells 16 after a predetermined period. Further, the ability of absorption of the drug 50 from the cellular apical membrane side to the cellular basement membrane side through the cultured cells 16 can be evaluated by adding the drug 50 to only the first liquid 38 out of the first liquid 38 and the second liquid 40 and then measuring the amount of the drug 50 in the second liquid 40 after a predetermined period. Further, the ability of excretion of the drug 50 from the cellular basement membrane side to the cellular apical membrane side through the cultured cells 16 can be evaluated by adding the drug 50 to only the second liquid 40 out of the first liquid 38 and the second liquid 40 and then measuring the amount of the drug 50 in the first liquid 38 after a predetermined period.

As described above, a monolayer film of cells that retains good physiological functions of the kidney and is substantially free of drug leakage can be obtained by adjusting the culture period of the cultured cells 16 on the base 12 to 12 hours or longer and 48 hours or shorter after seeding. Therefore, drug evaluation is preferably performed within a period of 12 hours or longer and 48 hours or shorter after seeding the cultured cells 16 on the base 12 and starting the culture.

### (Another Apparatus Using Cell Support Composite)

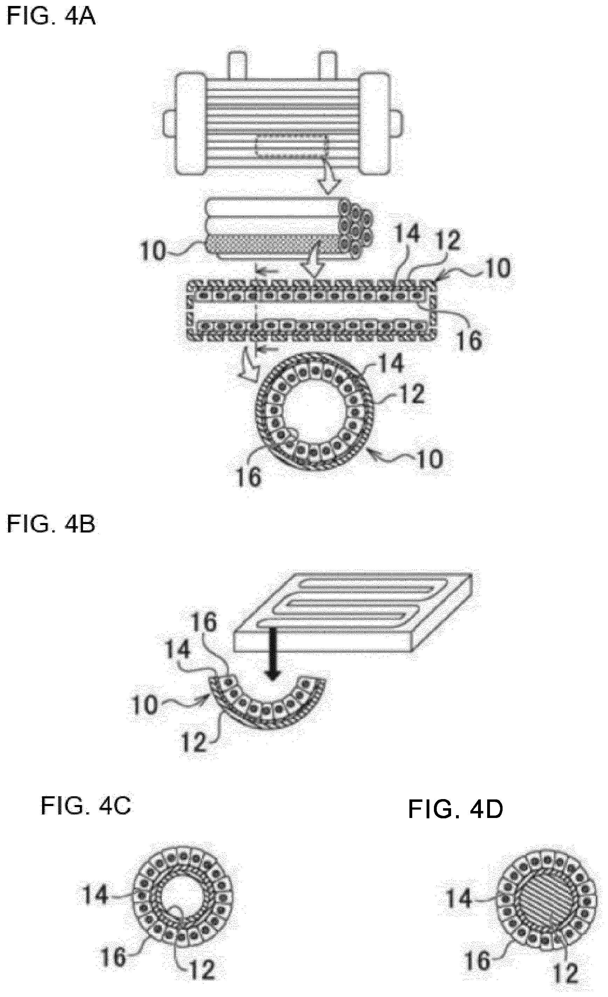

The cell support composite 10 can also be used for apparatuses other than Transwell described above. Fig. 4A to Fig. 4E are drawings schematically illustrating an adoption example of the cell support composite 10. Each of the drawing illustrates a part of a structure having incorporated therein the cell support composite 10.

Fig. 4A illustrates an apparatus having incorporated therein the cell support composite 10, in which a hollow fiber membrane is used as the base 12. In this apparatus, a monolayer film of cultured cells 16 is formed inside the tubular cavity of the hollow fiber membrane. Upon feeding of a liquid through the tubular cavity of the hollow fiber membrane, this apparatus allows the cultured cells 16 to incorporate a predetermined substance in this liquid and then transfer it out of the tubular cavity of the hollow fiber membrane. This apparatus can be used as, for example, a bioartificial kidney module that collects active substances from blood plasma components filtered through a blood filtering device.

Fig. 4B shows that the cell support composite 10 is incorporated into a micro flow channel chip. In the micro flow channel chip, the base 12 constitutes a micro flow channel. Furthermore, a monolayer film of cultured cells 16 is formed on the inner wall of the micro flow channel. In the micro flow channel chip, a very small amount of liquid flows within the flow channel, i.e., on the side where the cultured cells 16 are disposed. Then, a predetermined substance in the liquid is incorporated into the cultured cells 16. The micro flow channel chip can be used as, for example, a drug evaluation module for examining a function of a cell or intake and excretion of a drug with a very small amount of liquid. The micro flow channel chip may be used while being accommodated in a cartridge.

Fig. 4C shows that the cell support composite 10 constitutes a hollow microcarrier. In addition, Fig. 4D shows that the cell support composite 10 constitutes a solid microcarrier. In the hollow microcarrier and the solid microcarrier, the base 12 constitutes the carrier's main body. Furthermore, a monolayer film of cultured cells 16 is formed on the outer surface of the base 12. In the hollow microcarrier and the solid microcarrier, a very small amount of liquid flows on the side where the cultured cells 16 are disposed. Then, a predetermined substance in the liquid is incorporated into the cultured cells 16. Each of the microcarriers can be used as, for example, a drug evaluation module for examining a function of a cell or intake and excretion of a drug with a very small amount of liquid. Each of the microcarriers may be used while being accommodated in a cartridge.

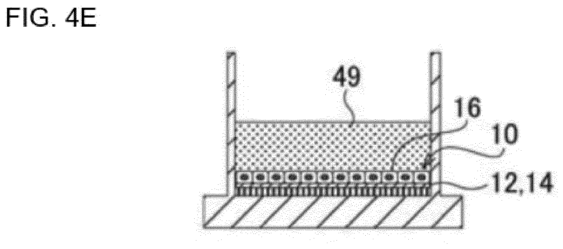

Fig. 4E shows that the cell support composite 10 is incorporated into a well bottom of a well plate. In a state where the cell support composite 10 is disposed at the well bottom, the cultured cells 16 face the upper side of the well. In the well plate, a very small amount of liquid 49 is injected into the well. Then, a predetermined substance in the liquid 49 is incorporated into the cultured cells 16. The well plate can be used as, for example, a drug evaluation module for examining a function of a cell or intake and excretion of a drug with a very small amount of liquid. The cell support composite 10 may alternatively be incorporated into a culture dish (such as a Petri dish) instead of the well plate.

As explained above, the method for producing the cell support composite 10 according to the present embodiment includes: culturing renal cells 24 in the aggregate 30 to prepare cultured cells 16, seeding these cultured cells 16 on the base 12 at a seeding density of 5×10⁴ cells/cm² or more and 18×10⁴ cells/cm² or less, and culturing the cultured cells on the base 12 for a period of 12 hours or longer and 48 hours or shorter to form a monolayer film of the cultured cells 16. As a result, the drug can be evaluated using a monolayer film of cultured cells 16 with improved physiological functions of the kidney and substantially free of drug leakage. Thus, the accuracy of the drug evaluation assay can be improved. In addition, the seeding density of 5×10⁴ cells/cm² or more and 12×10⁴ cells/cm² or less of the cultured cells 16 can easily extend the period during which the cell support composite 10 can be used for drug evaluation.

Further, the method for producing the drug evaluation apparatus 42 according to the present embodiment includes: installing the cell support composite 10 according to the present embodiment on the support portion 32 or preparing the cell support composite 10 in a state where the base 12 is installed on the support portion 32, thereby forming the first chamber 34 and the second chamber 36 that are aligned across the cell support composite 10, wherein at least one of the chambers contains the drug 50 to be evaluated. As a result, the drug evaluation apparatus 42 comprising the cell support composite 10, the support portion 32, the first chamber 34, and the second chamber 36, wherein at least one of the chambers contains the drug 50, can be obtained. Further, the drug evaluation method according to the present embodiment includes evaluating the transportation of the drug 50 through the cultured cells 16 using this drug evaluation apparatus 42. From the above, it is possible, according to the present embodiment, to perform membrane transport evaluation with higher accuracy using a Transwell, a common method of drug kinetic evaluation.

The embodiment of the present invention is described above in detail. The embodiment described above is merely a specific example of practicing the present invention. The details of the embodiment shall not be construed as limiting the technical scope of the present invention. A number of design modifications such as modification, addition, deletion, etc. of constituting elements may be made to the extent that they do not depart from the idea of the invention defined by the claims. New embodiments with design modifications will provide the combined advantages of the embodiment and the variation. Although the details subject to such design modification are emphasized in the embodiments by using phrases such as "of this embodiment" and "in this embodiment", details not referred to as such are also subject to design modification. Any combination of the above constituting elements is also useful as a mode of the present invention.

The embodiment may be defined by the following items.

### (Item 1)

A method for producing a cell support composite (10), including: culturing renal cells (24) in an aggregated state (30);
isolating the aggregate (30) into individual cultured cells (16);
seeding the cultured cells (16) on a base (12) at a seeding density of 5×10⁴ cells/cm² or more and 18×10⁴ cells/cm² or less; and
culturing the cultured cells (16) on the base (12) for a period of 12 hours or longer and 48 hours or shorter to form a monolayer film of the cultured cells (16).

### (Item 2)

The method for producing a cell support composite according to Item 1, wherein the seeding density is 5×10⁴ cells/cm² or more and 12×10⁴ cells/cm² or less.

### (Item 3)

A method for producing a drug evaluation apparatus (42) including:
placing the cell support composite (10) prepared by the method for producing a cell support composite (10) according to Item 1 or Item 2 on a support portion (32), or installing a base (12) on a support portion (32) and seeding cultured cells (16) on the base (12) by the method for producing a cell support composite (10) according to Item 1 or Item 2, thereby preparing a cell support composite (10); and
forming a first chamber (34) and a second chamber (36) that are aligned across the cell support composite (10), wherein at least one of the chambers contains a drug (50) to be evaluated.

### (Item 4)

A drug evaluation method, including evaluating transportation of a drug (50) by cultured cells (16) included in the cell support composite (10) using a drug evaluation apparatus (42) obtained by the method for producing a drug evaluation apparatus (42) according to Item 3.

### (Item 5)

A drug evaluation apparatus (42) including:
a cell support composite (10) produced by the method for producing a cell support composite (10) according to Item 1 or Item 2;
a support portion (32) that supports the cell support composite (10); and
a first chamber (34) and a second chamber (36) that are aligned across the cell support composite (10),
wherein at least one of the chambers contains a drug (50) to be evaluated.

### EXAMPLES

Hereinafter, an exemplary embodiment of the present invention will be described, but the exemplary embodiment is merely examples for suitably describing the present invention and shall not be interpreted as limiting the present invention in any way.

### (Analysis of Time-dependent Changes in Gene Expression Levels in Cells that Constitute Aggregate: Test 1)

First, 100,000 human renal proximal tubule epithelial cells (model number: CC-2553, Lonza Inc.) as an example of renal cells were seeded on a 60-mm Petri dish (Corning Inc.). A Petri dish having been subjected to adhesion treatment with coating with a gelatin solution (Sigma-Aldrich Corp.) was used. Then, the cells were cultured using REGM (Lonza, Inc.) as a culture solution under conditions of 37°C and 5% CO₂. Since the Petri dish was subjected to adhesion treatment, the cells did not aggregate during culture. Through two-dimensional culture in this Petri dish, renal proximal tubule epithelial cells with deteriorated physiological functions due to dedifferentiation were obtained.

A suspension of dedifferentiated cells was prepared. The cell concentration in the suspension was set to 10,000 cells/ml. The cell suspension was added dropwise on a 96-well U-bottom plate (Sumitomo Bakelite Co., Ltd.) that had been subjected to a treatment that makes the plate non-adherent to cells in an amount of 100 µl, and thereby cells were seeded thereon. Therefore, the seeding density was 1,000 cells/well. Then, the cells were cultured using REGM (model number: CC-3190, Lonza, Inc.) as a culture solution under conditions of 37°C and 5% CO₂. The culture solution was exchanged every two days. Through this culture, the cells formed aggregates.

mRNAs were extracted from cells immediately after seeding (that is, 0 hours) and cells that constitute aggregates on days 3, 4, 5, 6, 7, 8, 10, 12, and 14 (that is, 72, 96, 120, 144, 168, 192, 240, 288, and 336 hours) of culture using an RNeasy Mini Kit (QIAGEN NV) and then purified. Subsequently, cDNAs were synthesized from the purified mRNAs using a QuantiTect Reverse Transcription Kit (QIAGEN NV). These cDNAs were used as templates, and expression levels of the respective genes of AQP1, SGLT2, and OAT1 were measured by a real-time PCR method using Thermal Cycler Dice Real-Time System I (Takara Bio Inc.).

These genes are genes related to physiological functions of the kidney. Specifically, the AQP1 (aquaporin 1) gene is a gene encoding the AQP1 protein involved in transportation of water. The SGLT2 (sodium glucose cotransporter 2) gene is a gene encoding the SGLT2 protein involved in transportation of sodium and glucose. The OAT1 (organic anion transporter 1) gene is a gene encoding the OAT1 protein involved in transportation of drugs and, especially, transportation of compounds with a relatively low molecular weight.

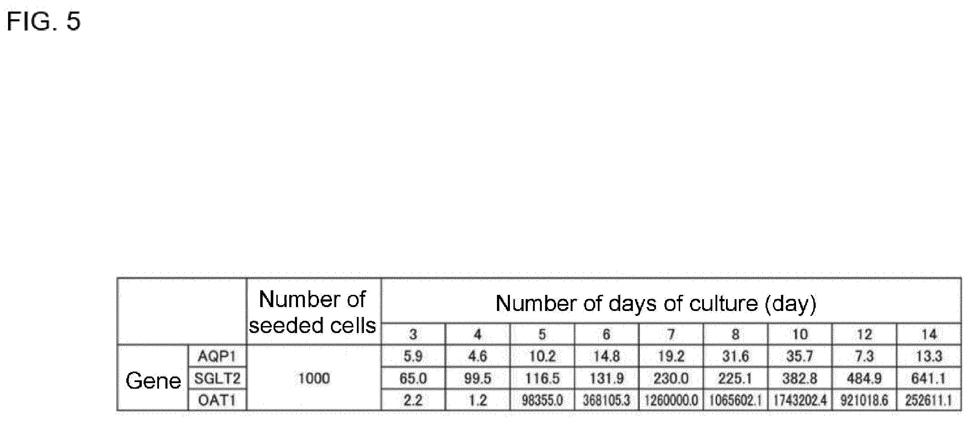

For each of the genes, the ratio (day M/day 0) of the expression levels on day M (M=3, 4, 5, 6, 7, 8, 10, 12, 14) of culture to the expression level immediately after seeding was calculated. Results are shown in Fig. 5. Fig. 5 is a chart summarizing time-dependent changes in gene expression levels in cells that constitute an aggregate. As summarized in Fig. 5, it was confirmed that the expression levels of each gene were increased by culturing human renal proximal tubule epithelial cells in an aggregated state. That is, it was confirmed that physiological functions of the cells were restored by agglutination culture. Especially, the expression level of the OAT1 gene was confirmed to be remarkably increased after 5 days or longer of culture.

The aggregates were also cultured for a prolonged period, and the expression levels of kidney-related genes in the cells that constituted the aggregates were analyzed over time. First, 1,000 dedifferentiated human renal proximal tubule epithelial cells (model number: CC-2553, Lonza, Inc.) were put into a 96-well plate that had been subjected to a treatment to make the plate non-adherent to cells and then cultured using 100 µl of REGM (model number: CC-3190, Lonza, Inc.) under conditions of 37°C and 5% CO₂. The medium was exchanged every two days. Through this culture, the cells formed aggregates.

mRNAs were extracted from cells on days 0, 4, 8, 10, 12, 14, 16, 20, 25, 30, 35, 40, 50, 60, 90 (that is, 0, 96, 192, 240, 288, 336, 384, 480, 600, 720, 840, 960, 1,200, 1,440, 2,160 hours) of culture using an RNeasy Mini Kit (QIAGEN NV) and then purified. Subsequently, cDNAs were synthesized from the purified mRNAs using a QuantiTect Multiplex RT-PCR Kit (QIAGEN NV).

These cDNAs were used as templates, and the expression levels of the respective genes of OAT1, OAT3 (organic anion transporter 3), OCT2 (organic cation transporter 2), MDR1 (multiple drug resistance 1), MATE1 (multidrug and toxin extrusion 1), MATE2 (multidrug and toxin extrusion 2), URAT1 (urate transporter 1), PEPT2 (peptide transporter 2), and SGLT2 were measured by the real-time PCR method using Thermal Cycler Dice Real-Time System I (Takara Bio Inc.). As a positive control, mRNA of renal cortex collected directly from the kidney of humans was used.

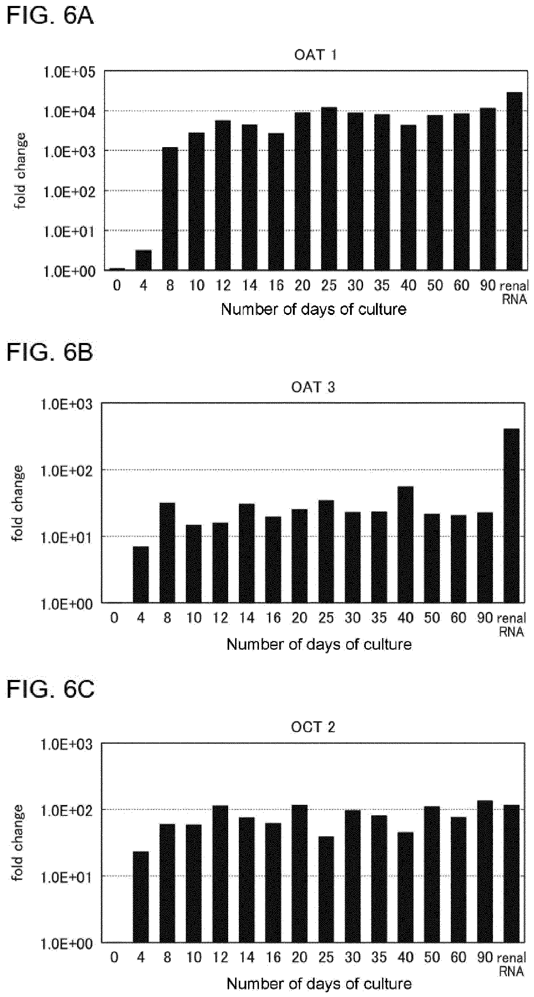

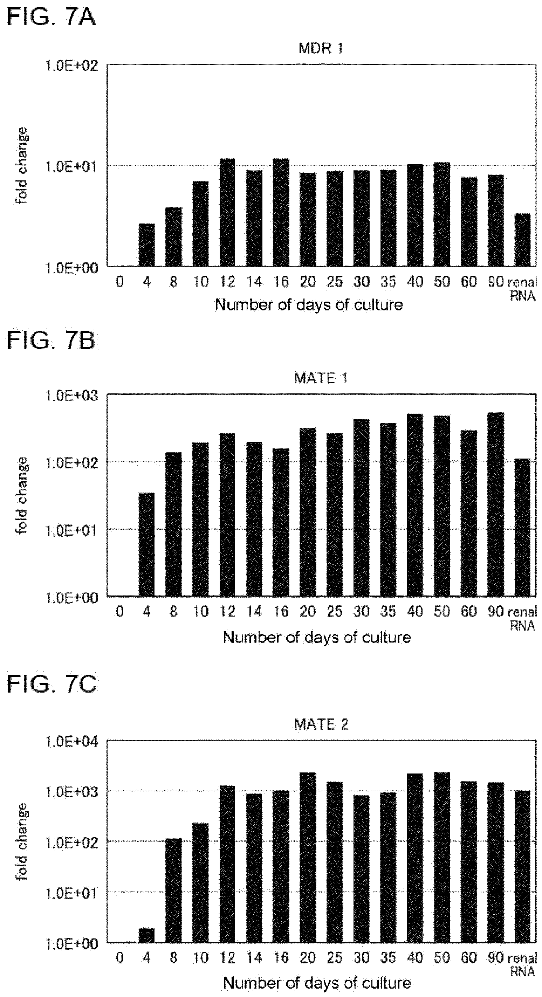

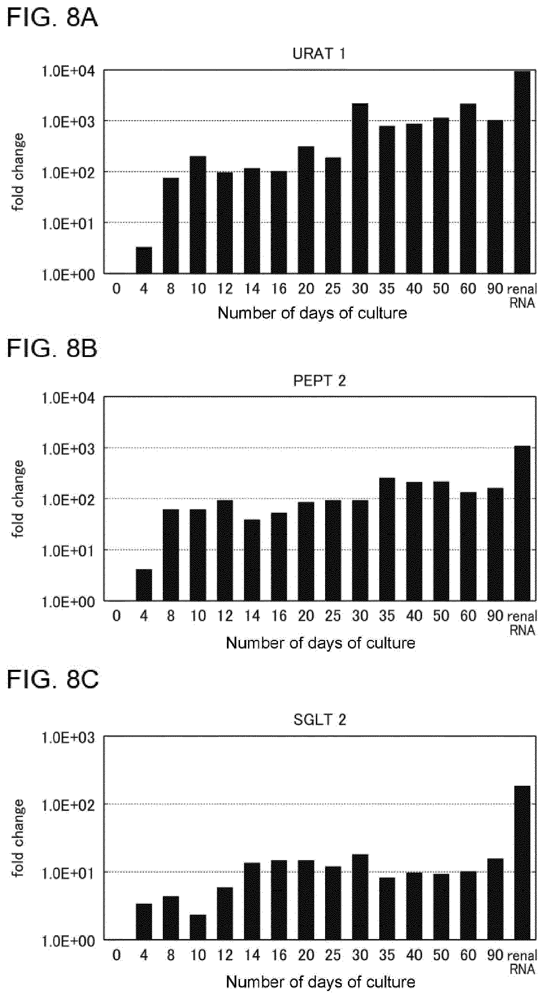

For each of the genes, the ratio (day M/day 0) of the expression levels on day M (M=4, 8, 10, 12, 14, 16, 20, 25, 30, 35, 40, 50, 60, 90) of culture to the expression level immediately after seeding was calculated. Results are illustrated in Fig. 6A, Fig. 6B, Fig. 6C, Fig. 7A, Fig. 7B, Fig. 7C, Fig. 8A, Fig. 8B, and Fig. 8C. Fig. 6A to Fig. 8C are graphs illustrating time-dependent changes in gene expression levels in cells that constitute aggregates.

As illustrated in Fig. 6A to Fig. 8C, it was confirmed that the expression levels of respective genes of OAT1, OAT3, OCT2, MDR1, MATE1, and MATE2, which encode drug transporter proteins involved in drug excretion from the vascular side to the tubule side, were increased by agglutination culture. It was also confirmed that the state of increased expression levels could be maintained for up to 90 days of culture. Similarly, it was confirmed that the expression levels of respective genes of URAT1, PEPT2, and SGLT2, which encode endogenous transporter proteins involved in the reabsorption of substances from the tubule side to the vascular side, were increased by aggregation culture, and that the state of increased expression levels could be maintained for up to 90 days of culture.

### (Isolation of Cells from Aggregate and Re-seeding: Test 2)

First, human renal proximal tubule epithelial cells (model number: CC-2553, Lonza, Inc.) were thawed and seeded on a Petri dish subjected to an adhesion treatment and were then cultured two-dimensionally using REGM (model number: CC-3190, Lonza, Inc.) under conditions of 37°C and 5% CO₂. The culture solution was exchanged every two days. Before the cells become confluent, Accutase (Innovative Cell Technologies, Inc.) was added to the Petri dish and reacted for 3 minutes at room temperature to retrieve the dedifferentiated cells. Then, a suspension of the cells was added dropwise on a 96-well V-bottom plate (PrimeSurface plate 96V, Sumitomo Bakelite Co., Ltd.) subjected to a treatment to make the plate non-adherent to cells, and thereby cells were seeded thereon. The seeding density was set to 1,000 cells/well. The cells were then cultured for 10 days under conditions of 37°C and 5% CO₂ using REGM. The culture solution was exchanged every two days. Through this culture, the cells formed aggregates.

After 10 days of culture, aggregates were retrieved and washed with PBS. Then, Accumax (Innovative Cell Technologies, Inc.) was added to the aggregates and reacted at 37°C for 10 minutes, and the aggregates were isolated into individual cells by pipetting. The reaction was continued for another 3 to 5 minutes to obtain a cell suspension in which almost all cells were isolated.

A Transwell provided with a cell culture insert (model number: 662-641, Greiner Bio-One) installed in each well of a 24-well plate (model number: 662-160, Greiner Bio-One) was prepared. The insert was prepared by adding dropwise a coating solution of 3.3 µL/well of laminin (iMatrix-511 silk, Nippi, Inc.) mixed with 100 µL/well of PBS on the day before use and allowing the mixture to stand overnight under refrigeration. The coating solution added dropwise on the insert was removed immediately before cell seeding, and the cells were re-seeded by adding dropwise the cell suspension on the insert. The amount of the cell suspension dropped was 150 µL/well. The seeding density was set to 4x10⁴ cells/well (12×10⁴ cells/cm²).

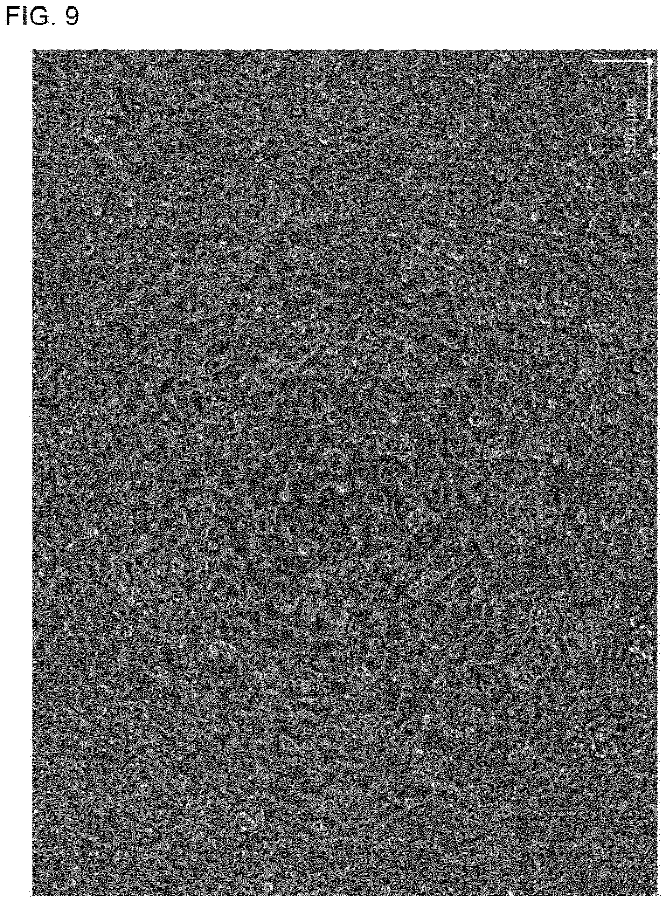

600 µL/well of REGM (Lonza, Inc.) was added to the well bottom of the Transwell (second chamber 36) to cultivate the cells. The culture solution was exchanged every day. Fig. 9 shows an optical microscope image of cells on the day after re-seeding (21 hours after re-seeding). Fig. 9 is an optical microscope image of cells isolated from an aggregate on the day after re-seeding. From Fig. 9, it was confirmed that enzymatic treatment of the aggregate can turn the aggregate into single cells, that resultant single cells can be uniformly dispersed on the insert, and that the cells can proliferate to a confluent state. That is, it was confirmed that a cell support composite can be formed using cells isolated from the aggregate.

### (Analysis of Changes Over Time in Monolayer Film: Test 3)

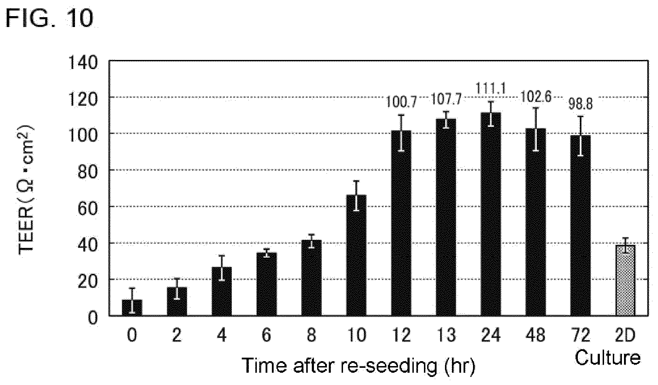

First, formation of aggregates, preparation of a cell suspension, and re-seeding of cells were performed in the same manner as in Test 2. The seeding density was set to 4x10⁴ cells/well (12×10⁴ cells/cm²). REGM (Lonza, Inc.) was added to the well bottom of the Transwell, and the cells were cultured. The culture solution was exchanged every day. After re-seeding the cells, the transepithelial electrical resistance (TEER) of the cells on the insert was measured over time using Millicell ERS-2 resistance measurement system (Millipore). Specifically, TEER was measured on the day of re-seeding until 13 hours after re-seeding, and every 24 hours from the day after re-seeding until the elapse of 72 hours. TEER is an electrical resistance value that is an indicator of formation of a monolayer of cells. When TEER is 100 Ωcm² or higher, it means that a uniform monolayer film with substantially no drug leakage is formed. Results are shown in Fig. 10.

Fig. 10 is a graph illustrating TEER at each elapsed time since re-seeding. As illustrated in Fig. 10, TEER was 100 Ωcm² or higher from 12 hours to 48 hours after re-seeding. Thus, it was confirmed that a monolayer film of cells with substantially no drug leakage can be obtained by adjusting the culture period of the cultured cells 16 on the base 12 to 12 hours or longer and 48 hours or shorter.

Fig. 10 also shows TEER of two-dimensionally cultured cells re-seeded on the insert (24 hours after re-seeding) as a reference. An apparently confluent monolayer was obtained also when two-dimensionally cultured cells were re-seeded. However, TEER was not increased to 100 Ωcm² or higher. This may be because physiological functions of the cells were not restored in the two-dimensional culture, and the expression levels of binding proteins that contribute to cell-to-cell binding did not increase, either. On the other hand, since the expression levels of binding proteins also increased in cells whose physiological functions were restored by aggregation culture, it is considered that a uniform monolayer film was formed, and TEER was increased to 100 Ωcm² or higher.

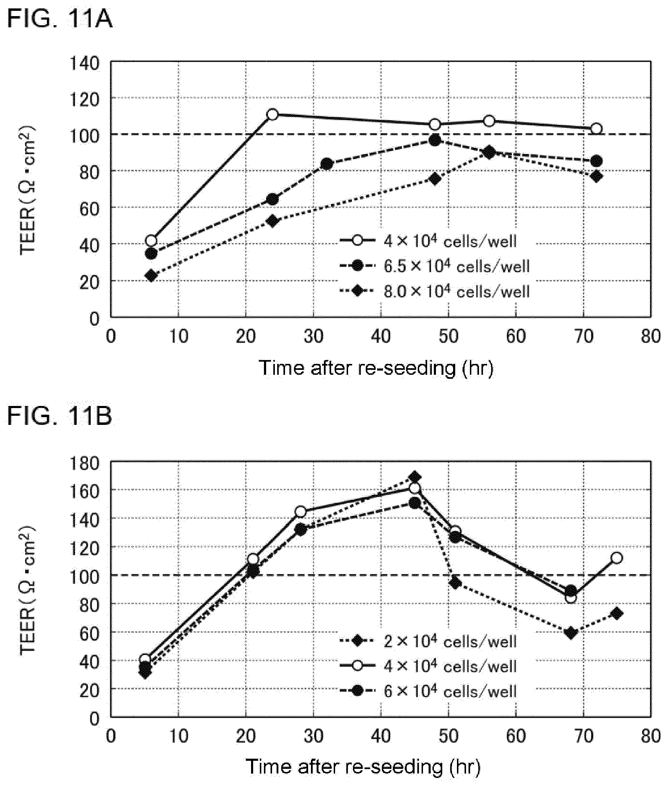

(Analysis of Relationship between Seeding Density and Monolayer Film: Test 4) First, formation of aggregates, preparation of a cell suspension, and re-seeding of cells were performed in the same manner as in Test 2. The seeding densities were set to 2×10⁴ cells/well (5×10⁴ cells/cm²), 4x10⁴ cells/well (12×10⁴ cells/cm²), 6×10⁴ cells/well (18×10⁴ cells/cm²), 6.5×10⁴ cells/well (19×10⁴ cells/cm²), 8×10⁴ cells/well (24x10⁴ cells/cm²). REGM (Lonza, Inc.) was added to the well bottom of the Transwell, and the cells were cultured. The culture solution was exchanged every day. After re-seeding the cells, TEER of the cells on the insert was measured over time using Millicell ERS-2 resistance measurement system (Millipore). Results are shown in Fig. 11A and Fig. 11B.

Fig. 11A and Fig. 11B are graphs illustrating time-dependent changes in TEER at each seeding density. As illustrated in Fig. 11A and Fig. 11B, TEER was not increased to 100 Ωcm² or higher in the case of a seeding density of 6.5×10⁴ cells/well (19×10⁴ cells/cm²) or more. On the other hand, TEER was increased to 100 Ωcm² or higher in the case of a seeding density of 2×10⁴ cells/well to 6×10⁴ cells/well (5×10⁴ cells/cm² to 18×10⁴ cells/cm²). Thus, it was confirmed that a monolayer film of cells with substantially no drug leakage was obtained by adjusting the seeding density of the cultured cells 16 on the base 12 to 5×10⁴ cells/cm² or more and 18×10⁴ cells/cm² or less.

Besides, in the case of a seeding density of 2×10⁴ cells/well to 4x10⁴ cells/well (5×10⁴ cells/cm² to 12×10⁴ cells/cm²), TEER was maintained at 100 Ωcm² or higher for a more prolonged period than the case of seeding densities of 6×10⁴ cells/well (18×10⁴ cells/cm²). Thus, it was confirmed that it is easier to extend the usable period of the cell support composite 10 or the drug evaluation apparatus 42 by adjusting the seeding density to 5×10⁴ cells/cm² or more and 12×10⁴ cells/cm² or less.

In Fig. 10, Fig. 11A, and Fig. 11B, TEERs at the seeding density of 4x10⁴ cells/well do not necessarily match. For example, in Fig. 11A, TEER is 100 Ωcm² or higher 21 hours after re-seeding. In Fig. 11B, TEER is 100 Ωcm² or higher 19 hours after re-seeding. This is presumably due to the different start times of TEER measurement. In addition, the maximum value of TEER is different between Fig. 11A and Fig. 11B. This is presumably because the TEER value tends to fluctuate depending on the measurement position in the well, the amount of culture solution in the well, the condition of the Transwell, and other factors. However, the present inventors have confirmed that TEER is 100 Ωcm² or higher with high reproducibility when the seeding density is 5×10⁴ cells/cm² or more and 18×10⁴ cells/cm² or less and when the culture period is 12 hours or longer and 48 hours or shorter.

### (Analysis of Changes in Gene Expression Levels in Cells after Reseeding Over Time: Test 5)

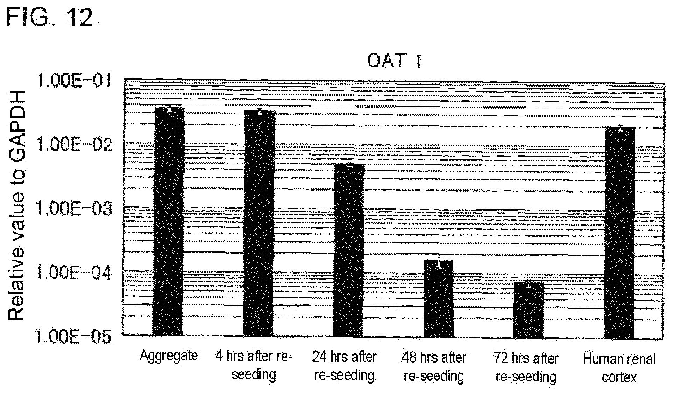

First, formation of aggregates, preparation of a cell suspension, and reseeding of cells were performed in the same manner as in Test 2. The seeding density was 4x10⁴/well (12×10⁴ cells/cm²). Cells were cultured by adding REGM (Lonza) to the bottom of the well of the Transwell. The culture medium changed daily. Cells on the insert were retrieved 0, 4, 24, 48, 72 hours after reseeding. Retrieval of cells 0 hours after reseeding is synonymous with retrieval of cells from aggregates. Using RNeasy Mini Kit (QIAGEN), mRNA was extracted from the retrieved cells and purified. Subsequently, cDNA was synthesized from the purified mRNA using a QuantiTect Whole Transcriptome Kit (QIAGEN). Using these cDNAs as a template, the expression level of the OAT1 gene was measured by real-time PCR using Thermal Cycler Dice Real Time System I (Takara Bio). Then, the value of the expression level of the OAT1 gene relative to the expression level of the GAPDH gene, which is a housekeeping gene, was calculated. In addition, the expression level of the OAT1 gene in the human renal cortex was also measured as a control, and the value relative to the expression level of the GAPDH gene was calculated. The results are shown in Fig. 12.

Fig. 12 is a diagram showing changes in the expression level of the OAT1 gene over time. As shown in Fig. 12, the expression level of the OAT1 gene decreased over time after reseeding. At 48 hours after reseeding, the expression level of the OAT1 gene decreased to about 1/100 of the expression of the OAT1 gene in aggregates and in the human renal cortex. In other words, from the viewpoint of gene expression level, it was confirmed that the physiological function required for drug evaluation was significantly reduced 48 hours after reseeding.

### (Analysis of Changes in Protein Expression Levels in Cells after Reseeding Over Time 1: Test 6)

Test 5 confirmed that genes related to the physiological function of the kidney disappeared 48 hours after reseeding to the extent that the cells were not suitable for drug evaluation. However, it can be said that, even if a gene related to the physiological function of the kidney is lost, the cell maintains a state suitable for use in drug evaluation if the expression of the protein encoded by the gene is maintained. In general, the expression level of the protein decreases with a delay from the decrease in the expression level of the gene. Therefore, the present inventor observed the expression of the OAT1 protein in cells after reseeding by immunocytostaining.

First, formation of aggregates, preparation of a cell suspension and reseeding of cells were performed in a manner similar to that of Test 2. The seeding density was 4x10⁴ cells/well (12×10⁴ cells/cm²). Cells were cultured by adding REGM (Lonza) to the bottom of the well of the Transwell. The culture medium was changed daily. In addition, a primary antibody solution obtained by diluting an OAT1 antibody (model number KE038, rabbit-derived, Transgenic) 50-fold with a blocking solution and a secondary antibody solution obtained by diluting an Alexa Fluor-labeled anti-rabbit antibody diluted 500-fold with a blocking solution were prepared.

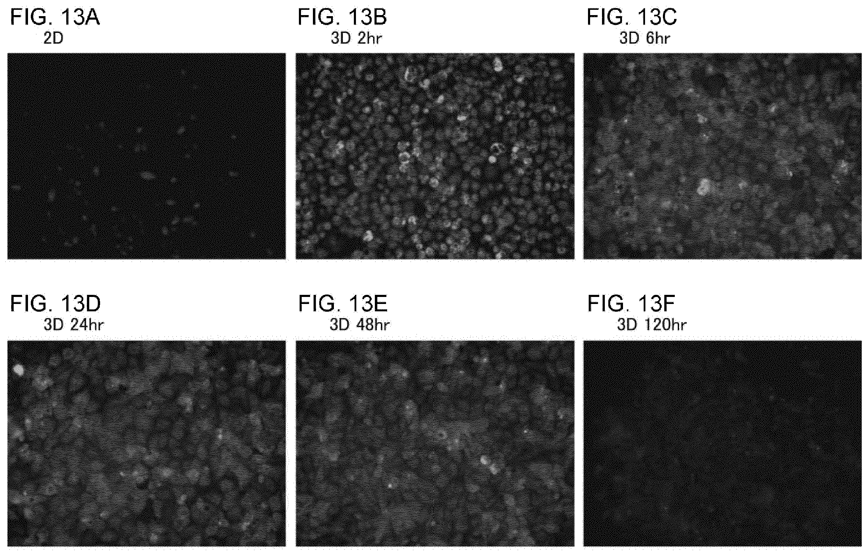

Cells 2, 6, 24, 48, 120 hours after reseeding were washed with PBS and fixed with a paraformaldehyde phosphate buffer (PFA). After fixation for more than 30 minutes, the cells were washed with PBS. PBS was replaced with a blocking solution and treated at room temperature for 1 hour. The blocking solution is a solution obtained by mixing donkey serum in 0.1% PBT to achieve a concentration of 5%. 0.1% PBT is a solution in which Triton X is added to PBS to achieve a concentration of 0.1%. Subsequently, the blocking solution was replaced with a primary antibody solution and treated overnight under refrigeration. Thereafter, the cells were washed three times with 0.1% PBT. Then, PBT was replaced with a secondary antibody solution and treated at room temperature for 1 hour. Nuclei were also stained using Hoechst 33342 for cell confirmation. Thereafter, the cells were washed three times with 0.1% PBT. PBT was replaced with PBS, and cells were observed using a fluorescence microscope (Keyence). The results are shown in Fig. 13A to Fig. 13F.

Fig. 13A to Fig. 13F are diagrams showing changes in the expression level of OAT1 protein over time. Fig. 13A is a fluorescence microscope image showing cells cultured in two dimensions and reseeded (24 hours after reseeding) as a reference. Fig. 13B to Fig. 13F are fluorescence microscope images obtained when cells cultured in aggregation culture are reseeded (2, 6, 24, 48, 120 hours after reseeding). As shown in Fig. 13A, expression of the OAT1 protein could not be observed in cells cultured in two dimensions. On the other hand, as shown in Fig. 13B to Fig 13F, expression of the OAT1 protein could be observed in cells cultured in aggregation culture until 48 hours after reseeding. In addition, the OAT1 protein was uniformly expressed on the insert. Therefore, it was confirmed that the cultured cells 16 retained the physiological function necessary for drug evaluation even 48 hours after reseeding.

### (Analysis of Changes in Protein Expression Levels in Cells after Reseeding Over Time 2: Test 6)

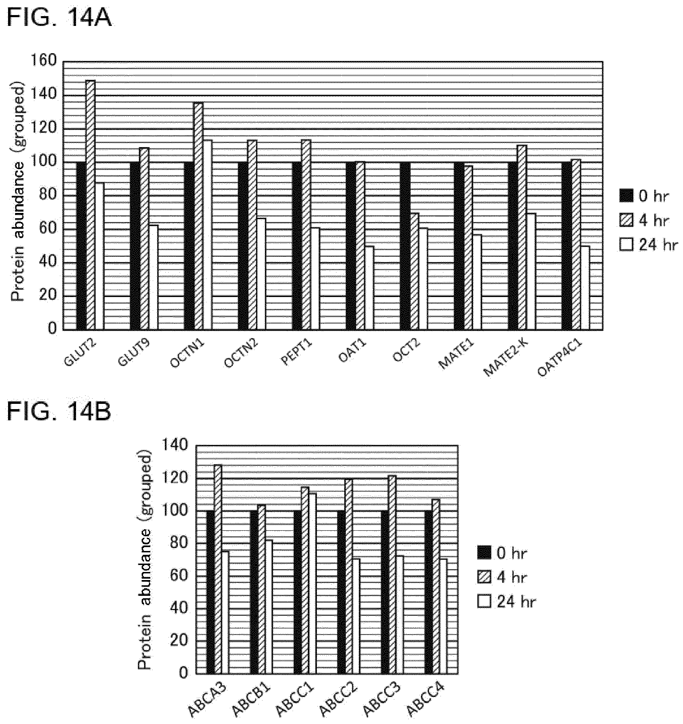

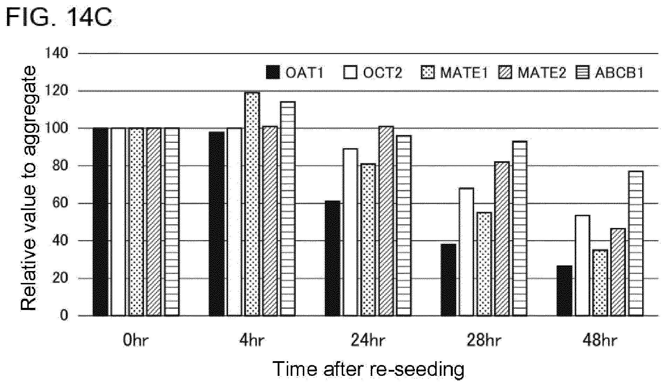

First, formation of aggregates, preparation of a cell suspension, and reseeding of cells were performed in the same manner as in Test 2. The seeding density was 4x10⁴ cells/well (12×10⁴ cells/cm²). Cells on the insert were retrieved 0, 4, 24, 28, 48 hours after reseeding. Proteins were extracted from the retrieved cells, and known proteomics analysis was performed using the extracted proteins. In the proteomics analysis, expression of all proteins (Total Lysate) and cell membrane proteins (Plasma membrane) of the retrieved cells was comprehensively analyzed. Then, from the results of proteomics analysis, the expression level of main transporter proteins of the kidney was calculated. The results are shown in Fig. 14A, Fig. 14B, and Fig. 14C.

Fig. 14A and Fig. 14B are diagrams showing the expression levels of kidney-associated transporter proteins in cell membrane proteins. Fig. 14C is a diagram showing changes over time in the expression levels of kidney-related transporter proteins in all proteins. Fig. 14A to Fig. 14C show the values relative to the expression levels of transporter proteins 0 hours after reseeding. Fig. 14A and Fig. 14B show the expression levels up to 24 hours after reseeding. Fig. 14C shows the expression levels up to 48 hours after reseeding.

Fig. 14A shows the expression levels of GLUT2 (glucose transporter 2), GLUT9 (glucose transporter 9), OCTN1 (organinc cation/carnitine transporter 1), OCTN2 (organinc cation/carnitine transporter 2), PEPT1 (peptide transporter 1), OAT1, OCT2, MATE1, MATE2-K (multidrug and toxin extrusion 2-K) and OATP4C1 (organic anion-transporting polypeptide 4C1), which are SLC transporter (solute carrier transporter) proteins of the intake system. Fig. 14B shows the expression levels of ABCA3, ABCB1 (MDR1), ABCC1, ABCC2, ABCC3 and ABCC4, which are ABC transporter (ATP Binding Cassette transporter) proteins in the excretion system. Fig. 14C shows the expression levels of OAT1, OCT2, MATE1, MATE2, and ABCB1 proteins.

As illustrated in Fig. 14A, the expression levels of the SLC transporter proteins were all maintained 40% or more in the cells after 24 hours from re-seeding. In addition, as illustrated in Fig. 14B, the expression levels of the ABC transporter proteins were all maintained 60% or more in the cells after 24 hours from re-seeding. Besides, as illustrated in Fig. 14C, the expression levels of the transporter proteins were all maintained 25% or more until 48 hours after re-seeding. When the expression levels of transporter proteins remain 20% or more relative to the expression levels in aggregates, then the cells are considered to maintain physiological functions required for drug evaluation. Thus, it was confirmed that the cultured cells 16 retain physiological functions necessary for drug evaluation even at 48 hours after re-seeding.

### (Membrane Transportation Evaluation Using Transwell: Test 7)

First, aggregates were formed, and a cell suspension was prepared from the aggregates to thereby re-seed the cells on the insert in the same manner as in Test 2. The seeding density was set to 4x10⁴ /well (12×10⁴ cells/cm²). Then, on the day after re-seeding (within 18 to 24 hours of re-seeding), membrane transport evaluation was performed using Transwell. In the membrane transport evaluation, radiolabeled Adefovir and radiolabeled Metformin were used. Adefovir is a drug as a substrate for OAT1, that is, a drug that is transported by OAT1. Metformin is a drug as a substrate for OCT2, that is, a drug that is transported by OCT2.

An assay buffer with each substrate added to a HBSS buffer was added to insert of the Transwell and treated for a predetermined time. For the Adefovir test, the treatment time was 2 minutes. For the Metformin test, the treatment time was 10 minutes. The cells were then retrieved, a cell suspension was prepared, and the radioisotopes in the cell suspension were quantified. As a result, the amount of substrate intake into the cell from the cellular apical membrane side was evaluated. The amount of substrate intake was also evaluated in the same manner when an inhibitor of OAT1, Probenecid, was added to the assay buffer and when an inhibitor of OCT2, Cimetidine, was added to the assay buffer.

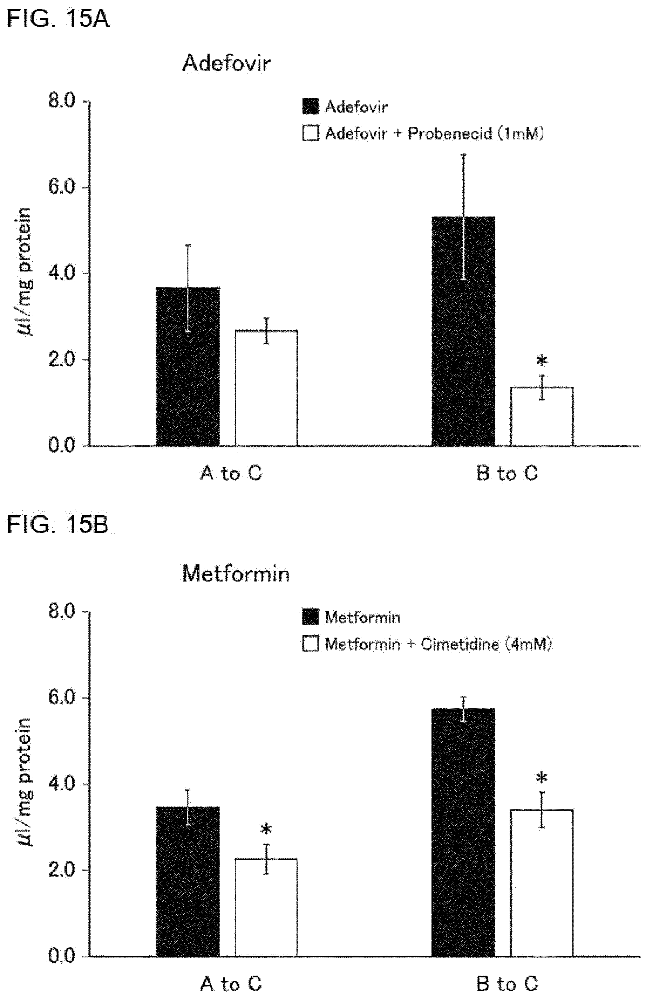

The assay buffer was also added to the well of the Transwell and treated for a predetermined time. For the Adefovir test, the treatment time was 2 minutes. For the Metformin test, the treatment time was 10 minutes. The cells were then retrieved, a cell suspension was prepared, and the radioisotopes in the cell suspensions were quantified. As a result, the amount of substrate intake into the cell from the cellular basement membrane side was evaluated. The amount of substrate intake was also evaluated in the same manner when an inhibitor Probenecid was added to the assay buffer and when an inhibitor Cimetidine was added to the assay buffer. Results are shown in Fig. 15A and Fig. 15B.

Fig. 15A is a graph illustrating results of membrane transportation of Adefovir. Fig. 15B is a graph illustrating results of membrane transportation of Metformin. In Fig. 15A and Fig. 15B, **"A to C"** indicates the amount of substrate intake into the cell from the cellular apical membrane side. "B to C" indicates the amount of substrate intake into the cell from the cellular basement membrane side.

As illustrated in Fig. 15A and Fig. 15B, both Adefovir and Metformin were confirmed to be taken into the cells. In addition, OAT1 and OCT2 are proteins that are expressed predominantly on the cellular basement membrane side. Therefore, for both Adefovir and Metformin, the amount of cellular intake into the cells was higher from the cellular basement membrane side than from the cellular apical membrane side. On the base, some cells may face the apical membrane sides and basement membrane sides in opposite directions. Therefore, substrate intake (A to C in the figure) in the case where the substrate is added to the insert was also observed.

Further, addition of each inhibitor significantly decreased the amount of substrate intake compared to the case of no addition. This confirmed that the intake of Adefovir was due to OAT1 and that of Metformin was due to OCT2. Based on the foregoing, it was confirmed that drug kinetic evaluation can be performed with high accuracy using the cell support composite 10 and the drug evaluation apparatus 42 according to the present embodiment.

### INDUSTRIAL APPLICABILITY

The present invention can be used in a method for producing a cell support composite, a method for producing a drug evaluation apparatus, a drug evaluation method, and a drug evaluation apparatus.

### REFERENCE SIGNS LIST

10 cell support composite, 12 base, 16 cultured cell, 24 renal cell, 30 aggregate, 32 support portion, 34 first chamber, 36 second chamber, 42 drug evaluation apparatus, 50 drug

## Claims

1. A method for producing a cell support composite, comprising:
culturing renal cells in an aggregated state;
isolating the aggregate into individual cultured cells;
seeding the cultured cells on a base at a seeding density of 5×10⁴ cells/cm² or more and 18×10⁴ cells/cm² or less; and
culturing the cultured cells on the base for a period of 12 hours or longer and 48 hours or shorter to form a monolayer film of the cultured cells.

2. The method for producing a cell support composite according to Claim 1,
wherein the seeding density is 5×10⁴ cells/cm² or more and 12×10⁴ cells/cm² or less.

3. A method for producing a drug evaluation apparatus, comprising:
placing the cell support composite prepared by the method for producing a cell support composite according to claim 1 or 2 on a support portion, or installing a base on a support portion and seeding cultured cells on the base by the method for producing a cell support composite according to claim 1 or 2, thereby preparing a cell support composite; and
forming a first chamber and a second chamber that are aligned across the cell support composite, wherein at least one of the chambers contains a drug to be evaluated.

4. A drug evaluation method, comprising
evaluating transportation of a drug by cultured cells included in the cell support composite using the drug evaluation apparatus obtained by the method for producing a drug evaluation apparatus according to claim 3.

5. A drug evaluation apparatus comprising:
a cell support composite produced by the method for producing a cell support composite according to claim 1 or 2;
a support portion that supports the cell support composite; and
a first chamber and a second chamber that are aligned across the cell support composite,
wherein at least one of the chambers contains a drug to be evaluated.
